Europäisches Patentamt

European Patent Office

Office européen des brevets

(19)

(11) Veröffentlichungsnummer: **0 281 678 B1**

(12) **EUROPÄISCHE PATENTSCHRIFT**

(45) Veröffentlichungstag der Patentschrift: **08.05.91**

(21) Anmeldenummer: **87118287.9**

(22) Anmeldetag: **10.12.87**

(51) Int. Cl.5: **C07G 17/00, A61K 35/10, C07C 37/60**

Die Akte enthält technische Angaben, die nach dem Eingang der Anmeldung eingereicht wurden und die nicht in dieser Patentschrift enthalten sind.

(54) **Verfahren zur Herstellung von niedermolekularen Alkalihuminaten.**

(30) Priorität: **12.03.87 DE 3707910**

(43) Veröffentlichungstag der Anmeldung:
**14.09.88 Patentblatt 88/37**

(45) Bekanntmachung des Hinweises auf die Patenterteilung:
**08.05.91 Patentblatt 91/19**

(84) Benannte Vertragsstaaten:
**AT BE CH DE ES FR GB GR IT LI LU NL SE**

(56) Entgegenhaltungen:

**LANTBRUKSHÖGSKOLANS ANNALER, Band 35, 1969, Seiten 815-822; I. LINDQUIST et al.: "On the formation of humic acids by oxidation of phenolic compounds"**

**CHIMIKA CHRONIKA, NEW SERIES, Band 1, 1972, Seiten 203-209; S. PARASKEWAS: "Über die Autoxydation von Hydrochinon in Alkalischen Lösungen Mittels Luftsauerstoff"**

**Chemical Abstracts 103: 98707, Plant and Soil 66, Seiten 205-215, 1982**

(73) Patentinhaber: **RÜTGERSWERKE AKTIENGESELLSCHAFT**
**Mainzer Landstrasse 217**
**W-6000 Frankfurt am Main 1(DE)**

(72) Erfinder: **Seubert, Bernhard**
**Meisenweg 14**
**W-6803 Edingen-Neckarhausen 1(DE)**
Erfinder: **Beilharz, Helmut, Dr.**
**Steinachstrasse 14**
**W-6905 Schriesheim(DE)**
Erfinder: **Fickert, Werner, Dr.**
**Stockacher Strasse 14**
**W-6800 Mannheim 61(DE)**
Erfinder: **Jeromin, Günter, Dr.**
**Bergastrasse 14**
**W-6900 Heidelberg(DE)**
Erfinder: **Spitaler, Ulrich, Dr.**
**Dürkheimer Hohl 2**
**W-6712 Freinsheim(DE)**

## Beschreibung

Die Erfindung betrifft die synthetische Herstellung von Alkalisalzen niedermolekularer Huminsäuren.

Huminsäuren oder allgemein Huminstoffen, werden heilende Wirkungen (vergleiché Ziechmann, Therapiewoche 28 (1978), 1199-1211), insbesondere auch wundheilende Wirkungen (vergl . C.A. 103:98707) zugesprochen. Andererseits sind die Huminsäuren bzw. ihre Salze parenteral als toxisch anzusehen (vergleiche Kühnert et al., Arch. exper. Vet. med. 36 (1982), 169-177), weshalb die Huminstoffe wohl, abgesehen von Moorbädern, vorwiegend in der Veterinärmedizin eingesetzt werden.

Es wurde gefunden, daß den Alkalisalzen niedermolekularer Huminsäuren eine Heilwirkung zukommt, die, im Gegensatz zu den bekannten Huminstoffen bzw. deren Salzen, wesentlich weniger toxisch ist.

Die niedermolekularen Huminstoffe, die Gegenstand der parallelen Anmeldung EP-A 0 281 679 der Anmelderin sind, sind zwar als Fraktion auch innerhalb der Bandbreite der natürlich vorkommenden Huminstoffe vorhanden, jedoch in relativ geringer Menge. Außerdem ist ihre Isolierung sehr aufwendig.

Es ist daher Aufgabe der Erfindung, ein einfaches synthetisches Verfahren bereitzustellen, bei dem diese niedermolekularen Huminsäuren in hoher Ausbeute anfallen und ohne großen Aufwand als Alkalisalze isoliert und als Heilmittel eingesetzt werden können.

Die Lösung der Aufgabe erfolgt durch ein Verfahren zur Herstellung der niedermolekularen Alkalihuminate gemäß der Ansprüche 1 bis 8, und ihre Verwendung nach den Ansprüchen 9 bis 11.

Es ist bekannt, daß man durch Sauerstoffoxidation von Phenolen, bevorzugt mehrwertigen Phenolen (Ziechmann, Huminstoffe, Verlag Chemie 1980, S. 260 ff. ) zu huminstoffähnlichen Produkten gelangen kann. So werden z. B. auch gemäß Lindquist, Lautbrukshögskolaus Annaler Bd. 35 (1969) 815-822 durch Luftoxidation von Pyrocatechol , Hydrochinon und Protocatechinsäure bei pH 5, 7 und 8 Huminsäuren hergestellt. Jedoch sind diese Produkte in vielerlei Hinsicht unterschiedlich zu den natürlichen Huminstoffen.

Es wurde nun gefunden, daß sich unter bestimmten alkalischen Bedingungen durch Oxidation von mehrwertigen Phenolen niedermolekulare Alkalihuminate herstellen lassen, die im wesentlichen, vor allem aber hinsichtlich ihrer Heilwirkung und ihrer geringen Toxität den natürlichen niedermolekularen Alkalihuminaten entsprechen.

Es ist dabei gleichermaßen überraschend, daß unter den gewählten Bedingungen einerseits die mehrwertigen Phenole quantitativ reagieren und daß andererseits die Umsetzung nicht zu hochmolekularen Produkten führt, sondern automatisch bei solchen Produkten zum Stillstand kommt, deren mittleres Molekulargewicht bei 1000 liegt bei einem Streubereich von 300 bis 1500, und die somit in die Kategorie der niedermolekularen Huminstoffe einzuordnen sind.

Ausgangsprodukte für das erfindungsgemäße Verfahren können alle gängigen mehrwertigen Phenole sein, wie Brenzkatechin, Resorcin, Hydrochinon, Orcin, Gallussäure, Protocatechusäure, Pyrogallol, 2-Oxyhydrochinon, Phloroglucin oder Tetraoxybenzole. Die mehrwertigen Phenole können als Reinsubstanzen oder im beliebigen Gemisch miteinander eingesetzt werden. Das bevorzugte mehrwertige Phenol ist Hydrochinon.

Zur Umsetzung werden die mehrwertigen Phenole in einer wäßrigen Alkalilauge gelöst. Als Alkalilaugen können alle Alkalimetallhydroxide verwendet werden. Aus wirtschaftlichen Gründen sind Natrium und Kaliumhydroxid bevorzugt. Entscheidend für die erfindungsgemäße Reaktion ist die Menge des eingesetzten Alkalihydroxids. Sie muß im Bereich der 1,4- bis 1,6-fach der stöchiometrischen Menge liegen, die zur Phenolatbildung der mehrwertigen Phenole, das heißt zur Neutralisation aller phenolischen OH-Gruppen notwendig ist. Dabei stellt sich ein pH-Wert der Reaktionslösung von 8,8 bis 9 ein.

Es ist einleuchtend, daß die eingesetzten Verbindungen möglichst rein sein sollen, denn dadurch werden unerwünschte Nebenreaktionen vermieden bzw. die erhaltenen Endprodukte lassen sich ohne weitere chemische Aufarbeitung - die auch mit unerwünschten chemischen Veränderungen der Endprodukte verbunden wären - erhalten und im erhaltenen Reaktionsmedium einsetzen.

Im allgemeinen ist es ausreichend, wenn als Wasser ein entmineralisiertes Wasser mit einer Leitfähigkeit von 6 bis 10 µS/cm und einem pH-Wert im Bereich von 5 bis 7 eingesetzt wird, als Alkalihydroxid entweder die Qualität "chemisch rein" oder der Reinheitgrad nach DAB 8.

Als mehrwertiges Phenol sollte ebenfalls eine "chemisch reine" Qualität eingesetzt werden mit einem Gehalt an mehrwertigem Phenol von mehr als 98 %.

Die Oxidation des mehrwertigen Phenolats kann entweder elektrochemisch oder plasmachemisch bzw. chemisch durch Durch- oder Überleiten von Sauerstoff oder eines sauerstoffhaltigen Gasgemisches erfolgen.

Die elektrochemische Oxidation erfolgt in einem elektrochemischen Reaktor, wobei die Oxidationsgeschwindigkeit durch Einstellung der Anodenspannung und der Stromdichte bestimmt wird.

Die Anodenspannung kamm im Bereich von 4 bis 15 V und die Stromdichte im Bereich von 0,5 bis 4 A/cm² variiert werden. Dabei liegt die Reaktionszeit im Bereich von 1 - 3 d.

Die plasmachemische Oxidation erfolgt in einer an sich bekannten Apperatur zur Coronaentladung, wobei die Oxidationsgeschwindigkeit durch Einstellung der Betriebsspannung und der Feldstärke bestimmt wird. Die Spannung kann im Bereich von 20 - 250 KV bei Frequenzen von 16 2/3 - 400 Hz und die Feldstärke von 80 KV/cm bis KV/cm variiert werden. Dabei liegt die Reaktionszeit im Bereich von 15 - 120 min.

Zur chemischen Oxidation wird die alkalische Lösung des mehrwertigen Phenolats in ein Reaktionsgefäß gebracht, das den unkontrollierten Zutritt von Luft verhindert, das heißt in ein geschlossenes Reaktionsgefäß, das aber mit einer Vorrichtung versehen ist, mittels der Gase ein- bzw. durchgeleitet werden können.

Nun wird unter Rühren Sauerstoff oder ein sauerstoffhaltiges Gasgemisch in ständigem Gasstrom entweder über oder durch die Reaktionslösung oder unter Druck auf die Reaktionslösung geleitet, wobei die Temperatur des Reaktionsgemisches im Bereich von 10 bis 40 °C, bevorzugt im Bereich von Raumtemperatur bis 30 °C liegt.

Als sauerstoffhaltiges Gasgemisch kann auch Luft eingesetzt werden, die aber vorher zur Absorption von $CO_2$ und zur Entfernung von Staubpartikeln über ein alkalisches Filter geleitet werden muß.

Diese Oxidationreaktion dauert, je nach gewählter Temperatur und Intensität der Sauerstoffzufuhr 5 bis 20 d. Während dieser Reaktionzeit färbt sich die Lösung intensiv dunkelbraun.

Die chemische Oxidation kann auch durch Reaktion mit milden Oxidationsmitteln wie Wasserstoffperoxid, seinen Anlagerungsverbindungen oder Persulfaten erfolgen.

Es ist ein Vorteil des erfindungsgemäßen Verfahrens, daß unter den gewählten Reaktionsbedingungen, ebenso wie bei der elektrochemischen Oxidation oder plasmachemischen auch bei der chemischen Oxidation die Bildungsreaktion der Huminstoffe nicht zu hochmolekularen Produkten führt, sondern bei Produkten mit einem mittleren Molekulargewicht von 1000, bei einem Streubereich von 300 bis 1500, von selbst zum Stillstand kommt. Dadurch erübrigt sich eine ständige Kontrolle über den Verlauf der Oxidationsreaktion und es ist nicht nachteilig, wenn die Reaktionsdauer länger ist, als es notwendig wäre.

Die aus der chemischen, plasmachemischen oder der elektrochemischen Oxidationsreaktion resultierende dunkelbraune Lösung wird nun neutralisiert und auf einen pH-Wert im Bereich von 6,2 bis 7,2, bevorzugt von 6,6 eingestellt und gepuffert. Dies erfolgt entweder durch Zugabe von Säure oder durch Einwirkung von saurem Ionenaustauscher und/oder nachfolgender Zugabe einer entsprechenden Pufferlösung (z.B. Phosphat-, Trisoder Zitronensäure- Puffer).

Sofern die neutralisierte und gepufferte Lösung unerwünschte Schwebstoffe enthält, wird sie nun durch ein Trennverfahren wie Zentrifugieren (10.000 bis 30.000×g) oder Filtieren durch ein sehr feinporiges Filtermaterial von diesen Schwebestoffen befreit. Obwohl diese Lösung für viele Anwendungszwecke direkt eingesetzt werden kann, sollte sie bei Verwendung der Produkte als Heilmittel durch an sich bekannte Reinigungsverfahren wie präparative Chromatographiemethoden, Ultrafiltration, Ultrazentrifugation oder Elektrodialyse gereinigt und von unerwünschten Nebenprodukten befreit werden.

Erhalten wird eine Lösung, die etwa 3 - 5 % niedermolekulare Alkalihuminate enthält. Diese Lösung kann direkt verwendet werden oder durch vorsichtiges Entfernen von Wasser, etwa mittels Gefriertrocknung zu einer 20 %igen Lösung aufkonzentriert werden.

Die erhaltene und die aufkonzentrierte Lösung sind stabil.

Bei Stabilitätskontrollen ist nach 60 Tagen Wechselbelastung 56/4 °C im 12/12 h Rhythmus keine Veränderung der Parameter Gehalt, pH-Wert, Redoxspannung und Mikrodialysetest über die Zufallsschwankungen hinaus feststellbar.

Toxizitätsprüfungen zeigen, daß die erhaltenen niedermolekularen Alkalihuminate gering toxisch sind, andererseits wurde gefunden, daß synthetische niedermolekulare Alkalihuminate ebenfalls die bei den natürlichen niedermolekularen Huminstoffen beobachtete Heilwirkung, insbesondere bei der Wundheilung zeigten.

Die erfindungsgemäß hergestellten niedermolekularen Alkalihuminate sind daher zur Herstellung von Heilmitteln insbesondere von Heilmitteln in der Wundheilung geeignet.

Durch die synthetische Herstellung der Alkalihuminate können Schwankungen der Zusammensetzung und des molekularen Aufbaus wie sie beim natürlichen Humifizierungsprozeß auftreten, weitgehend reduziert werden. Die erfindungsgemäßen niedermolekularen Alkalihuminate sind daher einheitlicher als entsprechende natürliche Produkte. Sie sind daher zur Herstellung standardisierter Heilmittel bestens geeignet. Insbesondere sind sie geeignet, stark schwankende Naturprodukte wie etwa Moor- oder Schlammbäder zu standardisieren, indem die erfindungsgemäßen Alkalihuminate zur Herstellung synthetischer Moorbäder verwendet werden. Dies kann entweder dadurch erfolgen, daß die synthetisch hergestellten niedermolekularen Alkalihuminate als hochwirksamer Moorbadersatz eingesetzt werden, oder daß sie natürlichen Moorbädern zugefügt werden.

Beispiel

In einem gegen Alkalien beständigen Rührwerkbehälter werden 3,2 kg Natronlauge (50 %ig, DAB 8) mit 100 l entmineralisiertem Wasser (pH 5 bis 7; Leitfähigkeit unter 5 $\mu$S/cm) vermischt.

Bei einer Temperatur im Bereich von 20 bis 30 °C werden im Verlauf von 45 min 5 kg Hydrochinon (chem.rein., über 99 %) zugegeben.

Die Apparatur wird nun verschlossen. Dann wird Luft, die über ein alkalisches Filtersystem von Staub, $CO_2$ und anderen Kontaminanten gereinigt wird, über die Oberfläche geleitet. Es wird 14 Tage lang gerührt, wobei eine Intervallschaltung, die alle 15 Minuten den Rührer für 3 - 5 min in Gang setzt, verwendet wird. Unter Aufnahme von Luftsauerstoff färbt sich die Lösung mit der Zeit intensiv dunkelbraun.

Die Reaktionstemperatur liegt im Bereich von 20 bis 25 °C.

Durch Zugabe von ortho-Phosphorsäure wird die Lösung nun auf pH 6,6 eingestellt und gleichzeitig gepuffert.

Mittels einer Vorfiltration durch ein feinporiges Filter (20 - 40 $\mu$m) werden Schwebstoffe von der Dialyseanlage ferngehalten.

Aus dieser Lösung werden in einer Anlage zur Elektrodialyse, die mit einer Membrane von 0,025 $\mu$m aus gesintertem Glas ausgestattet ist, durch Anlegen einer Gleichspannung von 200 V die erfindungsgemäßen Alkalihuminate als braune Lösung erhalten.

Das Retenat wird fortlaufend umgepumpt, um Konzentrationspolarisation zu vermeiden. Folgende Werte werden während der laufenden Dialyse kontrolliert:

pH-Wert : 6.4 bis 6,8
Mikrodialysetest : negativ

Aus der elektrophoretischen Wanderungsgeschwindigkeit wird für die so hergestellten Huminate ein mittleres Molekulargewicht von 1000 bei einem Streubereich von 300 bis 1500 ermittelt.

Toxität:

Bei Injektionen der 1 %igen Lösung an Versuchstieren (Mäuse) werden folgende Werte der LD $_{50}$ erhalten:

s.c. 1250 (mg/kg)
i.p. 810 (mg/kg)
i.v. 770 (mg/kg)

Stabilität:

Nach einer 6 monatigen Lagerung unter Luftabschluß bei 23 °C sind keine Veränderungen feststellbar.

Heilwirkung:

Eine nach Thrypsinbehandlung in Suspension gebrachte Kultur von L-Zellen (Mäusefibroblasten) wird mit 50 ppm niedermolekularem Alkalihuminat versetzt.

Die Kultur wird bei 37 °C unter Verwendung eines handelüblichen Nährmediums 48 h lang bebrütet.

Parallel hierzu wird als Vergleichsversuch eine analoge Kultur ohne Alkalihuminate gleichermaßen bebrütet. Danach wird die Anzahl der lebenden Zellen in beiden Kulturen bestimmt. In der mit niedermolekularen Alkalihuminaten versetzten Kultur liegt die Zahl der lebenden Zellen um 30 % höher als in der Vergleichskultur.

Wundheilung:

An 2 × 10 haarlosen Mäusen werden mit einem Mikrodermatom oberflächliche Wunden, die nur die obersten Epithelschichten erfaßten, in einer Größe von etwa 50mm$^2$ beigebracht.

Bei zehn dieser Mäuse wird die Wunde mit einer 1 %igen Lösung einmal benetzt. Die anderen Mäuse bleiben unbehandelt. Während des Beobachtungszeitraumes von 7 d läßt sich folgendes Beobachten:

Gegenüber den unbehandelten Mäusen nimmt bei den behandelten Versuchstieren

die Wundfläche rascher ab,
die Wunde trocknet früher ab,
die Granulation setzt früher ein und
die Wunde reinigt sich früher.

Insgesamt ist die Abheilung 2 - 3 d früher als bei den Kontrolltieren zu beobachten.

**Ansprüche**

1. Verfahren zur Herstellung von niedermolekularen Alkalihuminaten, **dadurch gekennzeichnet,** daß mehrwertige Phenole in schwach alkalischem wäßrigem Medium bei einer Temperatur im Bereich von 15 bis 40 °C oxidiert werden, danach die Reaktionsmischung auf einen pH-Wert im Bereich von 6,2 bis 7,2 eingestellt und/oder gepuffert wird und die niedermolekularen Alkalihuminate durch präparative Chromatographiemethoden, Ultrafiltration, Ultrazentrifugation oder Elektrodialyse gereinigt und von unerwünschten Nebenprodukten abgetrennt werden.

2. Verfahren nach Anspruch 1, **dadurch gekennzeichnet,** daß zum Herstellen der schwach alkalischen Reaktionslösung das mehrwertige Phenol mit der 1,4- bis 1,6-fachen stöchiometrischen Menge an Alkalilauge in Reaktion gebracht wird.

3. Verfahren nach Anspruch 1, **dadurch gekennzeichnet,** daß der pH-Wert der Reaktionsmischung im Bereich von 8,8 bis 9 liegt.

4. Verfahren nach den Ansprüchen 1 bis 3, **dadurch gekennzeichnet,** daß als mehrwertiges Phenol Hydrochinon eingesetzt wird.

5. Verfahren nach den Ansprüchen 1 bis 4, **dadurch gekennzeichnet,** daß die Oxidation durch Behandlung mit Sauerstoff oder einem sauerstoffhaltigen Gas erfolgt.

6. Verfahren nach den Ansprüchen 1 bis 4, **dadurch gekennzeichnet,** daß die Oxidation elektrochemisch erfolgt.

7. Verfahren nach den Ansprüchen 1 bis 4, **dadurch gekennzeichnet,** daß die Oxidation plasmachemisch erfolgt.

8. Verfahren nach den Ansprüchen 1 bis 4, **dadurch gekennzeichnet,** daß die Oxidation durch milde Oxidationsmittel erfolgt.

9. Niedermolekulare Alkali- oder Ammoniumsalze von Huminsäuren, hergestellt nach Anspruch 1, zur Verwendung als Wundheilmittel.

10. Verwendung der nach Anspruch 1 hergestellten niedermolekularen Alkali - oder Ammoniumsalze von Huminsäuren zur Herstellung von Heilmitteln in der Wundheilung.

11. Verwendung der nach Anspruch 1 hergestellten niedermolekularen Alkali- oder Ammoniumsalze zur Herstellung von synthetischen Moorbädern.

Patentansprüche für folgenden Vertragsstaat: ES

1. Verfahren zur Herstellung von niedermolekularen Alkalihuminaten, **dadurch gekennzeichnet,** daß mehrwertige Phenole in schwach alkalischem wäßrigem Medium bei einer Temperatur im Bereich von 15 bis 40 °C oxidiert werden, danach die Reaktionsmischung auf einen pH-Wert im Bereich von 6,2 bis 7,2 eingestellt und/oder gepuffert wird und die niedermolekularen Alkalihuminate durch präparative Chromatographiemethoden, Ultrafiltration, Ultrazentrifugation oder Elektrodialyse gereinigt und von unerwünschten Nebenprodukten abgetrennt werden.

2. Verfahren nach Anspruch 1, **dadurch gekennzeichnet,** daß zum Herstellen der schwach alkalischen Reaktionslösung das mehrwertige Phenol mit der 1,4- bis 1,6-fachen stöchiometrischen Menge an Alkalilauge in Reaktion gebracht wird.

3. Verfahren nach Anspruch 1, **dadurch gekennzeichnet,** daß der pH-Wert der Reaktionsmischung im Bereich von 8,8 bis 9 liegt.

4. Verfahren nach den Ansprüchen 1 bis 3, **dadurch gekennzeichnet,** daß als mehrwertiges Phenol Hydrochinon eingesetzt wird.

5. Verfahren nach den Ansprüchen 1 bis 4, **dadurch gekennzeichnet,** daß die Oxidation durch Behandlung mit Sauerstoff oder einem sauerstoffhaltigen Gas erfolgt.

6. Verfahren nach den Ansprüchen 1 bis 4, **dadurch gekennzeichnet,** daß die Oxidation elektrochemisch erfolgt.

7. Verfahren nach den Ansprüchen 1 bis 4, **dadurch gekennzeichnet,** daß die Oxidation plasmachemisch erfolgt.

8. Verfahren nach den Ansprüchen 1 bis 4, **dadurch gekennzeichnet,** daß die Oxidation durch milde Oxidationsmittel erfolgt.

9. Verwendung der nach den Ansprüchen 1-8 hergestellten niedermolekularen Alkalihuminate als Heilmittel.

10. Verwendung der nach den Ansprüchen 1-8 hergestellten niedermolekularen Alkalihuminate als Heilmittel in der Wundheilung.

11. Verwendung der nach den Ansprüchen 1-8 hergestellten niedermolekularen Alkalihuminate zur Herstellung von synthetischen Moorbädern.

**Claims**

1. A process for preparing low-molecular alkali huminates, characterized in that multivalent phenols are oxidized in a weakly alkaline aqueous medium at a temperature in the range of from 15 to 40°C, after which the reaction mixture is set at a pH value in the range of from 6.2 to 7.2 and/or is buffered and the low-molecular alkali huminates are purified by preparative chromatography methods, ultrafiltration, ultracentrifuging, or electrodialysis and are separated from undesired by-products.

2. A process according to Claim 1, characterized in that the multivalent phenol is reacted with a 1.4- to 1`6-fold stoichiometric quantity of alkali solution to prepare the weakly alkaline reaction solution.

3. A process according to Claim 1, characterized in that the pH value in the reaction mixture is in the range of from 8.8 to 9.

4. A process according to Claims 1 to 3, characterized in that hydroquinone is used as multivalent phenol.

5. A process according to Claims 1 to 4, characterized in that oxidation is performed by treatment with oxygen or an oxygen-containing gas.

6. A process according to Claims 1 to 4, characterized in that oxidation is performed electrochemically.

7. A process according to Claims 1 to 4, characterized in that oxidation is performed plasmachemically.

8. A process according to Claims 1 to 4, characterized in that oxidation is performed by mild oxidizing agents.

9. Low-molecular alkali or ammonium salts of humic acids, produced according to Claim 1, for use as wound healing agents.

10. Use of the low-molecular alkali or ammonium salts of humic acids produced according to Claim 1 for the preparation of wound healing agents.

11. Use of the low-molecular alkali or ammonium salts produced according to Claim 1 for the preparation of synthetic mud baths.

Claims for the following Contracting State: ES

---

1. A process for preparing low-molecular alkali huminates, characterized in that multivalent phenols are oxidized in a weakly alkaline aqueous medium at a temperature in the range of from 15 to 40°C, after which the reaction mixture is set at a pH value in the range of from 6`2 to 7`2 and/or is buffered and the low-molecular alkali huminates are purified by preparative chromatography methods, ultrafiltration, ultracentrifuging, or electrodialysis and are separated from undesired by-products.

2. A process according to Claim 1, characterized in that the multivalent phenol is reacted with a 1.4- to 1.6-fold stoichiometric quantity of alkali solution to prepare the weakly alkaline reaction solution.

3. A process according to Claim 1, characterized in that the pH value in the reaction mixture is in the range of from 8.8 to 9.

4. A process according to Claims 1 to 3, characterized in that hydroquinone is used as multivalent phenol.

5. A process according to Claims 1 to 4, characterized in that oxidation is performed by treatment with oxygen or an oxygen-containing gas.

6. A process according to Claims 1 to 4, characterized in that oxidation is performed electrochemically.

7. A process according to Claims 1 to 4, characterized in that oxidation is performed plasmachemically.

8. A process according to Claims 1 to 4, characterized in that oxidation is performed by mild oxidizing agents.

9. Use of the low-molecular alkali huminates produced according to Claims 1 to 8 as healing agents.

10. Use of the low-molecular alkali huminates produced according to Claims 1 to 8 as healing agents in healing wounds.

11. Use of the low-molecular alkali huminates produced according to Claims 1 to 8 for the preparation of synthetic mud baths.

**Revendications**

1. Procédé de préparation d'humates alcalins de bas poids moléculaire, caractérisé en ce que des polyphénols sont oxydés en milieu aqueux faiblement alcalin à une température comprise dans la gamme de 15 à 40°C, qu'ensuite le mélange réactionnel est ajusté à une valeur de pH dans le domaine de 6,2 à 7,2 et/ou tamponné et que les humates alcalins de bas poids moléculaire sont purifiés par des méthodes de chromatographie préparative, par ultrafiltration, ultracentrifugation ou électrodialyse et séparés des produits secondaires indésirables.

2. Procédé selon la revendication 1, caractérisé en ce que, pour la préparation de la solution réactionnelle faiblement alcaline, le polyphénol est mis à réagir avec une quantité 1,4 à 1,6 fois stoechiométrique de lessive alcaline.

3. Procédé selon la revendication 1, caractérisé en ce que la valeur de pH du mélange réactionnel se situe dans la gamme de 8,8 à 9.

4. Procédé selon les revendications 1 à 3, caractérisé en ce que de l'hydroquinone est mise en oeuvre en tant que polyphénol.

5. Procédé selon les revendications 1 à 4, caractérisé en ce que l'oxydation s'effectue par traitement avec de l'oxygene ou avec un gaz contenant de l'oxygène.

6. Procédé selon les revendications 1 à 4, caractérisé en ce que l'oxydation s'effectue par voie électrochimique.

7. Procédé selon les revendications 1 à 4, caractérisé en ce que l'oxydation s'effectue par voie plasmachimique.

8. Procédé selon les revendications 1 à 4, caractérisé en ce que l'oxydation s'effectue à l'aide d'agents d'oxydation doux.

9. Sels alcalins ou d'ammonium d'acides humiques de bas poids moléculaire, préparés selon la revendication 1, pour utilisation en tant qu'agents cicatrisants.

10. Utilisation des sels alcalins ou d'ammonium d'acides humiques de bas poids moléculaire préparés selon la revendication 1 pour l'élaboration de médicaments pour la cicatrisation des plaies.

11. Utilisation des sels alcalins ou d'ammonium d'acides humiques de bas poids moléculaire préparés selon la revendication 1 pour la préparation de bains de boue synthétiques.

Revendication pour l'Etat contractant suivant: ES

1. Procédé de préparation d'humates alcalins de bas poids moléculaire, caractérisé en ce que des polyphénols sont oxydés en milieu aqueux faiblement alcalin à une température comprise dans la gamme de 15 à 40°C, qu'ensuite le mélange réactionnel est ajusté à une valeur de pH dans le domaine de 6,2 à 7,2 et/ou tamponné et que les humates alcalins de bas poids moléculaire sont purifiés par des méthodes de chromatographie préparative, par ultrafiltration, ultracentrifugation ou électrodialyse et séparés des produits secondaires indésirables.

2. Procédé selon la revendication 1, caractérisé en ce que, pour la préparation de la solution réactionnelle faiblement alcaline, le polyphénol est mis à réagir avec une quantité 1,4 à 1,6 fois stoechiométrique de lessive alcaline.

3. Procédé selon la revendication 1, caractérisé en ce que la valeur de pH du mélange réactionnel se situe dans la gamme de 8,8 à 9.

4. Procédé selon les revendications 1 à 3, caractérisé en ce que de l'hydroquinone est mise en oeuvre en tant que polyphénol.

5. Procédé selon les revendications 1 à 4, caractérisé en ce que l'oxydation s'effectue par traitement avec de l'oxygéne ou avec un gaz contenant de l'oxygène.

6. Procédé selon les revendications 1 à 4, caractérisé en ce que l'oxydation s'effectue par voie électrochimique.

7. Procédé selon les revendications 1 à 4, caractérisé en ce que l'oxydation s'effectue par voie plasmachimique.

8. Procédé selon les revendications 1 à 4, caractérisé en ce que l'oxydation s'effectue à l'aide d'agents d'oxydation doux.

9. Utilisation des humates alcalins de bas poids moléculaire préparés selon les revendications 1 à 8 en tant que médicaments.

10. Utilisation des humates alcalins de bas poids moléculaire préparés selon les revendications 1 à 8 en tant que médicaments pour la cicatrisation des plaies.

11. Utilisation des humates alcalins de bas poids moléculaire préparés selon les revendications 1 à 8 pour la préparation de bains de boue synthétiques.